# EUROPEAN PATENT APPLICATION

(11) **EP 2 813 246 A1**
(43) Date of publication of application: **17.12.2014**
(21) Application number: 13171346.3
(22) Date of filing: 11.06.2013
(51) Int. Cl.: A61K 49/18, G01N 24/08, G01N 33/58, C12Q 1/37

(54) **Compositions for detecting analytes by magnetic resonance imaging**

(71) Applicant: Albert-Ludwigs-Universität Freiburg, 79085 Freiburg (DE)
(72) Inventor: Shastri, Prasad V., 79112 FREIBURG (DE); Wyss, Pradeep P., 79104 FREIBURG (DE)
(74) Representative: Dr. Langfinger & Partner

(57) **Abstract**

Injectable compositions for the MRI detection of an analyte selected from the group consisting of reactive oxygen species, proteases and enzymes, comprising
a) a matrix material based on a responsive hydrophobic polymer capable of undergoing a chemical reaction with the analyte to be detected, such reaction leading to a disruption of the polymer chain of the responsive polymer,
b) a contrast agent suitable for use in magnetic resonance imaging, embedded in or encapsulated in the polymer a),
c) optionally, a functionality capable of binding a marker or probe or a probe for creating a second detection signal, and
d) optionally, a non-responsive polymer not undergoing a chemical reaction with the analyte under the conditions where polymer a) undergoes a reaction leading to chain breakage

## Description

The present invention relates to novel compositions for the detection of analytes by magnetic resonance imaging.

Magnetic resonance imaging (MR I) has become one of the most important diagnosis tools available in medicine. While typically MRI is not capable of sensing biochemical activities, the appearance of activated MRI contrast agents, whose relaxing is variable in response to a specific parameter change in the surrounding physiological microenvironment potentially allows for MRI to monitor biological processes.

An ideal bioanalytical sensor should achieve, in life cells and in vivo, real-time tracking of biological or chemical or physical processes as well as detection of disease-related abnormal features with little or no interferences.

Until today, fluorescent molecular probes have been playing a major role in the development of such intracellular sensing and imaging devices. However, these molecular probes have several drawbacks affecting the reliable measurement. The probe molecules have to be in a cell permeable form, which often requires proper derivatization of the molecules, which in itself might interfere with their function.

Furthermore, the cytotoxicity of the available dyes is often a problem as the mere presence of these molecules may interfere with the processes to be detected.

The size of classical molecular probes often constitutes another problem in introducing the probe into the organism where the process to be monitored takes place.

These problems have partly been solved by the development of nanoparticles available as platforms for constructing new types of sensors which, due to their small particle size, are easier to introduce into the systems. A single spherical nanoparticle of approximately 500 nm in diameter is considerably smaller in volume than typical mammalian cell and nanoparticles available today with sizes of 150 nm or less even better minimize the interference due to physical size. Most of the nanoparticles are non-toxic and therefore do not cause chemical interference to cells either.

Polymeric nanoparticles, in many cases based on poly(D,L-lactic-coglycolic acid) or polycaprolactone have been widely used for the delivery of therapeutics such as small molecular drugs, nucleic acids peptides and proteins.

Yang et al., Biomaterials 30 (2009), 3882-3890 discloses super paramagnetic iron oxide nanoparticles encapsulated in microbubbles as dual contrast agents of magnetic resonance and ultrasound imaging. According to figure 1 of this document the iron oxide nanoparticles are distributed in a layer of a polylactide polymer which is surrounded by a shell of poly vinyl alcohol.

Murthy et al., Nature materials 6 (2007), 765-769 discloses the in vivo imaging of hydrogen peroxide with chemiluminescent nanoparticles. These nanoparticles comprise a hydrophobic polymer containing peroxalate ester groups in its backbone in which a fluorescent dye is embedded. Hydrogen peroxide reacts with the oxalate ester groups in the polymer chain, thereby leading to a cleavage of the polymer chain and the creation of an instable intermediate species which transfers energy to the fluorescent dye when decaying , which in return provides a fluorescence signal which can be determined by classical fluorescence spectroscopy. The method depends on the energy transfer, i.e. the interaction of the metabolite of the reaction between analyte and polymer, with the fluorescent dye.

There has been very limited development of systems for MRI detection which change their magnetic properties in response to enzymatic or chemical species, especially within a tissue environment.

A galactopyranose modified Gd-chelate compound where the chelate is a tetraazamacrocycle has been described by Meade, Acc. Chem. Res. 2009, 42(7): 893-903 and in Meade et al., J. Angew. Chem. 36, 726-728 (1997).

Magnetic resonance imaging (MRI) has received increasing attention in the recent past for this purpose because it is non-invasive and provides information about biological structure and function of whole organisms over time.

It is generally desirable to monitor species in an organism which are associated to the development of diseases or in general indicative of abnormal conditions.

Detection and monitoring of such analytes through non-invasive technologies capable of delivering structural information would be especially desirable.

**Description of the figures**

Figure 1 shows the TEM image of ultra small superparamagnetic iron oxide (USPIO) particles used in the working examples.

Figure 2 shows the particle size distribution of the superparamagnetic iron oxide nanoparticles used in the working examples

Figure 3 shows a TEM image of the superparamagnetic iron oxide particles encapsulated in a peroxalate functionalized copolymer.

Figure 4 shows the ¹H-NMR spectra of superparamagnetic iron oxide nanoparticles dissolved in CHCl₃/CDCl₃ at various concentrations_{.}

Figure 5 shows the correlation of T2 relaxation time and concentration for superparamagnetic iron oxide particles used in the working examples.

Figure 6 shows the changes in relaxation time for different concentrations of iron oxide encapsulated in peroxalate polymer.

Figure 7 shows the change in relaxation time for a composition in accordance with the present invention with and without the addition of hydrogen peroxide as analyte.

Figure 8 shows the change of relaxation time over time in a system comprising hydrogen peroxide as analyte.

It was an object of the present invention to develop compositions for the detection of analytes selected from reactive oxygen species, proteases and enzymes through magnetic resonance imaging (MRI).

This object is achieved through the compositions in accordance with claim 1.

Preferred embodiments of the present invention are set forth in the dependent claims and in the detailed specification hereinafter.

The injectable compositions in accordance with the present invention comprise as component a) a matrix material based on a responsive hydrophobic polymer capable of undergoing a chemical reaction with the analyte to be detected, such reaction leading to a disruption of the polymer chain of the responsive polymer.

Injectable, as used herein, is intended to denote the capability of applying the compositions by injection into the system where the analyte is to be detected. The skilled person is aware that such compositions have to fulfil certain requirements concerning viscosity and solvents used and will select the appropriate conditions.

So called "responsive" or "stimulus-responsive" polymers are capable of exhibiting reversible or irreversible changes in physical properties and-or chemical structures in response to small changes in external environment.

Responsive polymers can be generally categorized into two main types of the basis of detection principles, namely molecular recognition-based sensors and chemical reaction-based ones.

Chemical reaction based responsive polymers rely on the change of polymer properties through chemical reaction whereas molecular-recognition based sensors do not necessarily require chemical reactions.

The responsive hydrophobic polymers used in the compositions of the present invention are chemical-reaction based responsive polymers. The analyte to be detected reacts with the responsive polymer. The reaction leads to a disruption of the polymer chain thereby reducing the molecular weight and changing physical and chemical properties of the polymer.

The common feature of the chemical-reaction based responsive polymers in accordance with the present invention is the presence of a suitable functionality in the chain which reacts with the analyte to be detected upon injection of the composition in accordance with the present invention into the system. It is apparent that the nature of the functionality depends on the analyte to be detected, i.e. the skilled person will tailor the polymer through use of suitable monomers depending on the analyte to be detected. The functionality might thus be different for different analytes to be detected.

The choice of the functionality also determines the selectivity of the probe for a given analyte. The more precisely the functionality is tailored to the specific analyte, the better the selectivity will be and will thus allow selective monitoring of different analytes.

The amount of functional groups providing the desired reactivity with the analyte in the polymer chain can vary over a broad range. The reaction of the analyte with the functional unit in the polymer chain breaks the chain and creates at least two reaction products of lower molecular weight which can give rise to a change in the relaxation of water molecules in the vicinity of the reaction and/or which can induce or lead to an agglomeration of a contrast agent. Both effects yield a signal which is detectable by magnetic resonance imaging.

A first group of responsive polymers in the compositions in accordance with the present invention are so called peroxalate polymers, i.e. responsive polymers which comprise units in the polymer chain formally derived from oxalic acid. This group of polymers is in particular suitable for the determination of reactive oxygen species (ROS) which will be described in more detail hereinafter.

Peroxalate polymers thus generally comprise the structural element

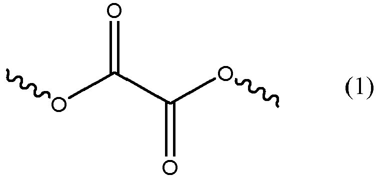

in the polymer chain.

The synthesis of peroxalate based polymers has been described in the literature, i.a. in the reference to Murthy et al. discussed hereinbefore. The polymers disclosed in Murthy are also suitable for the compositions of the present invention. Thus, suitable polymers can e.g. be obtained by reacting oxalyl chloride with suitable comonomers as described in this reference. Additional information on peroxalate polymers is also provided in the examples hereinafter. It has been shown that polyoxalate and copolyoxalates have smooth tissue interaction and can be used for controlled release. Since the polyoxalates can be synthesized using hydrophobic diols, the polymer can be formulated into nanoparticles via nanoprecipitation. The degradation products of these polymers are very biocompatible.

Another group of responsive polymers are groups having elements

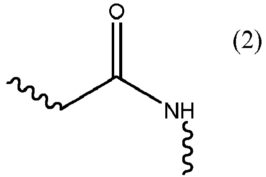

in the polymer chain.

This group of polymers has been found particularly useful for the detection of proteases as analytes, which will also be described in more detail hereinafter.

This kind of functional groups in the polymer chain can be obtained by reacting a diacid with a diamine, a reaction well known to the skilled person, so that no further details need to be given here. If diacids are reacted with diamines, polymers are obtained which have repeating units represented by the above structure.

If other monomers, not providing the required functionality, are included in the monomer mixture, the degree of units of formula (1) and/or (2) can be adjusted to the desired range for a specific application and copolymers are obtained which comprise the units of formula (1) and/or (2) in the chain. The nature of the other monomers not providing the desired functionality is not critical and can be selected from any group of monomer capable of undergoing a copolymerization reaction with the monomers providing the desired functionality.

Alternatively, multifunctional polymerizable monomers can be used which have already groups of formulae (1) or (2) in the molecule, and, in addition to the group of formula (1) or (2) comprise at least two polymerizable groups. In the course of the polymerization, the groups of formula (1) or (2) are then incorporated into the polymer chain and thus provide the desired functionality in the chain to react with the analyte to be detected. Again, as above, these monomers can be copolymerized with other monomers not providing the desired functionality.

The amount of elements providing the desired functionality influences the detection limit of the analyte to a certain degree and thus the skilled person can use this for adjusting the detection limit for a given analyte in accordance with the specific situation.

From the foregoing it is apparent that a variety of responsive polymers are suitable for use in the compositions in accordance with the present invention.

The injectable compositions of the present invention are suitable to detect analytes selected from reactive oxygen species, proteases and enzymes.

Reactive oxygen species (ROS) is a collective term for small chemically reactive molecules containing oxygen. Examples include oxygen ions and peroxides. ROS are involved in ROS form as a natural byproduct of the normal metabolism of oxygen and have important roles in cell signaling and homeostasis. ROS are involved in signalling pathways that maintain cellular homeostasis in physiological processes. A balanced production of ROS is crucial for normal aerobic metabolism and running several signalling pathways in the body. However, during times of environmental stress (e.g., UV or heat exposure), ROS levels can increase dramatically. This may result in significant damage to cell structures. Cumulatively, this is known as oxidative stress or oxidative injury. Overproduction of ROS leads to oxidative stress and the accumulation of oxidative stress-induced damage over time is associated with a number of different diseases, such as cancer.

ROS are also generated by exogenous sources such as ionizing radiation affecting biological systems.

Effects of ROS on cell metabolism are well documented in a variety of species. These include not only roles in apoptosis (programmed cell death) but also positive effects such as the induction of host defence genes and mobilisation of ion transport *systems.* This implicates them in control of cellular function. In particular, platelets involved in wound repair and blood homeostasis release ROS to recruit additional platelets to sites of injury. These also provide a link to the adaptive immune system via the recruitment of leukocytes.

Reactive oxygen species are implicated in cellular activity to a variety of inflammatory responses including cardiovascular disease. They may also be involved in hearing impairment via cochlear damage induced by elevated sound levels, in ototoxicity of drugs such as cisplatin, and in congenital deafness in both animals and humans. ROS are also implicated in mediation of apoptosis or programmed cell death and ischaemic injury. Specific examples include stroke and heart attack.

In aerobic organisms the energy needed to fuel biological functions is produced in the mitochondria via the electron transport chain. In addition to energy, reactive oxygen species (ROS) with the potential to cause cellular damage are produced. ROS can damage DNA, RNA, and proteins, which, in theory, contributes to the physiology of ageing.

ROS are produced as a normal product of cellular metabolism. One major contributor to oxidative damage is hydrogen peroxide (H₂O₂), which is converted from superoxide that leaks from the mitochondria. Catalase and superoxide dismutase ameliorate the damaging effects of hydrogen peroxide and superoxide, respectively, by converting these compounds into oxygen and water, benign molecules. However, this conversion is not 100% efficient, and residual peroxides persist in the cell. While ROS are produced as a product of normal cellular functioning, excessive amounts cause deleterious effects. Memory capabilities decline with age, evident in human degenerative diseases such as Alzheimer's disease, which is accompanied by an accumulation of oxidative damage.

Accumulation of ROS can decrease an organism's fitness because oxidative damage is a contributor to senescence. In particular, the accumulation of oxidative damage may lead to cognitive dysfunction.

Accumulating oxidative damage can affect the efficiency of mitochondria and further increase the rate of ROS production. The accumulation of oxidative damage and its implications for aging depends on the particular tissue type where the damage is occurring. It has also been suggested that oxidative damage is responsible for age-related decline in brain functioning. This has led to the conclusion that oxidation of cellular proteins is potentially important for brain function.

Exogenous ROS can be produced from pollutants, tobacco, smoke, drugs, xenobiotics, or radiation.

Ionizing radiation can generate damaging intermediates through the interaction with water, a process termed radiolysis. Since water comprises 55-60% of the human body, the probability of radiolysis is quite high under the presence of ionizing radiation. In the process, water loses an electron and become highly reactive. Then through a three-step chain reaction, water is sequentially converted to hydroxyl radical (-OH), hydrogen peroxide (H2O2), superoxide radical (02-) and ultimately oxygen (02). The hydroxyl radical is extremely reactive that immediately removes electrons from any molecule in its path, turning that molecule into a free radical and so propagating a chain reaction. But hydrogen peroxide is actually more damaging to DNA than hydroxyl radical since the lower reactivity of hydrogen peroxide provides enough time for the molecule to travel into the nucleus of the cell, subsequently wreaking havoc on macromolecules such as DNA.

ROS are constantly generated and eliminated in the biological system and are required to drive regulatory pathways. Under normal physiologic conditions, cells control ROS levels by balancing the generation of ROS with their elimination by scavenging system. But under oxidative stress conditions, excessive ROS can damage cellular proteins, lipids and DNA, leading to fatal lesions in cell that contribute to carcinogenesis.

Cancer cells exhibit greater ROS stress than normal cells do, partly due to oncogenic stimulation, increased metabolic activity and mitochondrial malfunction. At low levels, ROS facilitates cancer cell survival since cell-cycle progression driven by growth factors and receptor tyrosine kinases (RTK) require ROS for activation and chronic inflammation, a major mediator of cancer, is regulated by ROS. A high level of ROS can suppress tumor growth through the sustained activation of cell-cycle inhibitor and induction of cell death as well as senescence by damaging macromolecules. In fact, most of the chemotherapeutic and radiotherapeutic agents kill cancer cells by augmenting ROS stress. The ability of cancer cells to distinguish between ROS as a survival or apoptotic signal is controlled by the dosage, duration, type, and site of ROS production. Modest levels of ROS are required for cancer cells to survive, whereas excessive levels kill them.

Most risk factors associated with cancer interact with cells through the generation of ROS. ROS then activate various transcription factors such as nuclear factor kappa-light-chain-enhancer of activated B cells (NF-κB), activator protein-1 (AP-1), hypoxia-inducible factor-1α and signal transducer and activator of transcription 3 (STAT3), leading to expression of proteins that control inflammation; cellular transformation; tumor cell survival; tumor cell proliferation; and invasion, angiogenesis as well as metastasis. And ROS also control the expression of various tumor supressor genes such as p53, retinoblastoma gene (Rb), and phosphatase and tensin homolog (PTEN).

A cancer cell can die in three ways: apoptosis, necrosis and authophagy. Excessive ROS can induce apoptosis through both the extrinsic and intrinsic pathways. An even higher ROS level can result in both apoptosis and necrosis in cancer cells. ROS can also induce cell death through autophagy, which is a self-catabolic process involving sequestration of cytoplasmic contents (exhausted organelles and protein aggregates) for degradation in lyposomes.

From the foregoing it is apparent that the monitoring of ROS in a tissue environment, i.e. in vivo can assists in diagnosis and treatment of a variety of diseases and thus the compositions of the present invention which are capable to detect reactive oxygen species in the low concentrations in which they are normally present in vivo can be successfully used in these applications.

Proteases are a second group of analytes which can be detected with the compositions of the present invention.

A protease (also termed peptidase or proteinase) is any enzyme that conducts proteolysis, that is, begins protein catabolism by hydrolysis of the peptide bonds that link amino acids together in the polypeptide chain forming the protein.

Proteases occur naturally in all organisms. These enzymes are involved in a multitude of physiological reactions from simple digestion of food proteins to highly regulated cascades (e.g., the blood-clotting cascade, the complement system, apoptosis pathways, and the invertebrate prophenoloxidase-activating cascade). Proteases can either break specific peptide bonds (limited proteolysis), depending on the amino acid sequence of a protein, or break down a complete peptide to amino acids (unlimited proteolysis). The activity can be a destructive change, abolishing a protein's function or digesting it to its principal components; it can be an activation of a function, or it can be a signal in a signaling pathway.

Proteases are sometimes also referred to as proteolytic enzymes. They belong to the class of enzymes known as hydrolases, which catalyse the reaction of hydrolysis of various bonds with the participation of a water molecule.

Proteases are divided into four major groups according to the character of their catalytic active site and conditions of action: serine proteinases, cysteine (thiol) proteinases, aspartic proteinases, and metalloproteinases. Attachment of a protease to a certain group depends on the structure of catalytic site and the amino acid (as one of the constituents) essential for its activity.

Proteases are used throughout an organism for various metabolic processes. Acid proteases secreted into the stomach (such as pepsin) and serine proteases present in duodenum (trypsin and chymotrypsin) enable us to digest the protein in food; proteases present in blood serum (thrombin, plasmin, Hageman factor, etc.) play important role in blood-clotting, as well as lysis of the clots, and the correct action of the immune system. Other proteases are present in leukocytes (elastase, cathepsin G) and play several different roles in metabolic control. Proteases determine the lifetime of other proteins playing important physiological role like hormones, antibodies, or other enzymes-this is one of the fastest "switching on" and "switching off" regulatory mechanisms in the physiology of an organism. By complex cooperative action the proteases may proceed as cascade reactions, which result in rapid and efficient amplification of an organism's response to a physiological signal.

Cathepsins may be mentioned as a first exemplary group of proteases which can be detected and monitored with the compositions of the present invention.

Cathepsins are proteases (enzymes that degrades proteins) found in all animals as well as other organisms. There are approximately a dozen members of this family, which are distinguished by their structure, catalytic mechanism, and which proteins they cleave. Most of the members become activated at the low pH found in lysosomes. Thus, the activity of this family lies almost entirely within those organelles. There are, however, exceptions such as cathepsin K, which works extracellularly after secretion by osteoclasts in bone resorption.

Cathepsins have a vital role in mammalian cellular turnover, e.g. bone resorption. They degrade polypeptides and are distinguished by their substrate specificities.

Cathepsins are indicative of a variety of disease conditions.

Deficiencies in cathepsin A are linked to multiple forms of galactosialidosis. The cathepsin A activity in lysates of metastatic lesions of malignant melanoma is significantly higher than in primary focus lysates. Cathepsin A increased in muscles moderately affected by muscular dystrophy and denervating diseases.

Cathepsin B was found to break down the amyloid plaques, the root of Alzheimers symptoma. Cathepsin B has also been implicated in the progression of various human tumors including ovarian cancer. Cathepsin B is also involved in apoptosis as well as degradation of myofibrillar proteins in myocardial infarction.

Cathepsin K is involved in osteoporosis, a disease in which a decrease in bone density causes an increased risk for fracture.

Thus, it is apparent that monitoring and detecting cathepsins in vivo can help in the diagnosis and treatment of a variety of abnormal conditions and diseases. The compositions of the present invention are capable of detecting cathepsins in the low concentration in which they are occurring in vivo through magnetic resonance imaging.

Another exemplary group of proteases which can be detected with the compositions of the present invention through magnetic resonance imaging are the matrix metalloproteases (MMPs).

Matrix metalloproteases are zinc-dependent endopeptidases and belong to the greater group of a family of proteases known as metzincin superfamily.

Collectively, they are capable of degrading all kinds of extracellular matrix proteins, but also can process a number of bioactive molecules. They are known to be involved in the cleavage of cell surface receptors, the release of apoptotic ligands (such as the FAS ligand), and chemokine/cytokine in/activation. MMPs are also thought to play a major role on cell behaviors such as cell proliferation, migration (adhesion/dispersion), differentiation, angiogenesis, apoptosis, and host defense.

The compositions of the present invention can also be used to monitor and detect enzymes through magnetic resonance imaging.

Enzymes are highly selective catalysts, greatly accelerating both the rate and specificity of metabolic reactions, from the digestion of food to the synthesis of DNA. Most enzymes are proteins, although some catalytic RNA molecules have been identified. Enzymes adopt a specific three-dimensional structure, and may employ organic (e.g. biotin) and inorganic (e.g. magnesium ion) cofactors to assist in catalysis.

Since enzymes are selective for their substrates and speed up only a few reactions from among many possibilities, the set of enzymes made in a cell determines which metabolic pathways occur in that cell.

Enzymes serve a wide variety of functions inside living organisms. They are indispensable for signal transduction and cell regulation, often via kinases and phosphatases. Viruses can also contain enzymes for infecting cells, such as the HIV integrase and reverse transcriptase, or for viral release from cells, like the influenza virus neuraminidase.

Since the tight control of enzyme activity is essential for homeostasis, any malfunction (mutation, overproduction, underproduction or deletion) of a single critical enzyme can lead to a genetic disease. The importance of enzymes is shown by the fact that a lethal illness can be caused by the malfunction of just one type of enzyme out of the thousands of types present in our bodies.

From the foregoing it is apparent that the three types of analytes which are monitored and/or detected with the compositions of the present invention through magnetic resonance imaging are indicative of a great variety of diseases or abnormal conditions and thus their monitoring, in particular in vivo, which is possible with the compositions of the present invention, is important in the diagnosis of diseases.

Magnetic resonance imaging (MRI) is one of the most powerful, non-invasive diagnostic imaging modalities in medicine and biomedical research for its superior resolution and for providing in-depth anatomical details in the early diagnosis of many diseases as well as for gaining information on the anatomy, function and metabolism of tissues in vivo. In MRI, the nuclear spin of water protons, which are abundant in the body, is manipulated by external magnetic fields to produce images. In the magnetisation of water protons, the longitudinal *T1* and transverse *T2* relaxation times, whose values are tissue dependent, are important in the generation of contrast.

Apart from differences in the local water content, the basic contrast in the MR image mainly results from regional differences in the intrinsic relaxation times T1 and T2, each of which can be independently chosen to dominate image contrast. However, the intrinsic contrast provided by the relaxation T1 and T2 of the hydrogen atoms in the water molecules and changes in their values brought about by tissue pathology are often too limited to enable a sensitive and specific diagnosis. For that reason increasing use is made of MRI contrast agents that alter the image contrast following intravenous injection. The degree and location of the contrast changes provide substantial diagnostic information.

T1 contrast agents enhance images by decreasing the longitudinal relaxation time through interaction of a paramagnetic atom with surrounding water protons. The most common paramagnetic atom used in this regard is gadolinium and thus a significant number of T1 contrast agents are based on Gd³⁺.

The most widely used T2 shortening agents (shortening the transverse relaxation time T2) are based on iron oxide particles and are often referred to as superparamagnetic iron oxide (SPIO).

Depending on their chemical composition, molecular structure and overall size, the in vivo distribution volume and pharmacokinetic properties vary between different contrast agents which can be used to tailor same to specific diagnostic tests. The skilled person will select the appropriate contrast agent tailored to the specific situation.

Both groups of contrast agents (affecting T1 and/or T2 relaxation) can be used in the compositions of the present invention and the skilled person will select the best suitable contrast agent for a specific application based on his professional experience and knowledge.

The analytes react with the functional elements in the matrix polymer of the compositions of the present invention which results in chain breakage of the polymer. This chain breakage can lead to a variety of effects which are susceptible to magnetic resonance imaging. First, the chain breakage and the molecular weight change associated therewith can modify the water uptake behaviour of the polymer which will lead to a change of the relaxation of water molecules present in the system which is then detected with magnetic resonance imaging.

The principle may be explained for oxalate polymers in the detection of hydrogen peroxide as a representative of a reactive oxygen species as an example as follows:

Peroxalate integrated into a polymer structure will create a compound degradable to hydrogen peroxide. If SPIO particles are encapsulated in polymeric peroxalate functionalized nanoparticles, they will be degraded by hydrogen peroxide. Due to this degradation various effects occur at the same time, which all have influence on the relaxation behavior of the water protons. As the polymer matrix degrades, water is penetrating more into the nanoparticle and the distance from the encapsulated SPIO's to the water starts getting smaller, and therefore the influence of the magnetic fields increases on the nearby protons. The magnetite nanoparticles are not soluble in water and therefore form aggregates. This will change the magnetic moment of the magnetite and also decrease the metal surface which limits the area where protons can relax faster, and thus leads to a relaxation change. The relaxation changes can be followed by measuring the T2 relaxation with NMR spectroscopy

MRI contrast agents for in vivo MRI measurements should be biocompatible, easily dispersible and stable in diverse local *in vivo* environments; should easily penetrate the tissue and selectively target the biomarker of interest (present in low concentrations); should be capable of amplifying the signal enhancement in order to generate a sufficient image contrast; should have sufficiently long circulation times in the blood; and should be safely cleared from the body.

Micro- or nanoparticulate contrast agents are particularly suitable as component b) of the compositions of the present invention. Microparticulate, as used herein, is intended to denote an weight average mean diameter of more than 300 nm but less than 500 µm, whereas the term nanoparticulate denotes particles having a weight average mean diameter of 300 nm or less, in particular 200 nm or less and particularly preferred 150 nm or less. Weight average mean diameter may be determined by light scattering or by size exclusion chromatography. Respective methods have been described in the literature so that no further details need to be given here.

Engineered nanoparticulate contrast agents are valuable and potentially transformative tools for enhancing clinical diagnostics for a wide range of *in vivo* imaging modalities including MRI of cancer or neurodegenerative diseases.

Nanoparticulate MRI contrast agents greatly improve the sensitivity of MRI as they contain a high amount of contrast-generating material, and due to their small size they can easily penetrate into tissue. Being a nanomaterial, the contrast agents have a large surface area for the incorporation of functional groups (to improve targeting efficacy), tunable circulation half-life, and the potential to function as both targeted imaging and drug delivery (i.e., 'theranostic') agents. For molecular targeted MRI, a variety of different targeting ligands can be conjugated on surfaces of these nanoparticles. The targeting ligands include monoclonal antibodies, antibody fragments, peptidomimetics, small peptides and recombinant proteins.

Superparamagnetic iron oxide (SPIO) may be mentioned as a first group of suitable contrast agents for the compositions of the present invention. The size of the iron oxide particles may be used to control and adjust their physiochemical and pharmacokinetic properties.

SPIO contrast agents enhance T₁ as well as - predominantly - T₂/T₂* relaxation. Because of its significant impact on the reduction of the transversal relaxation times, T₂/T₂* is mostly used in MR imaging as a negative contrast means.

The general crystal structure of iron oxide is Fe₂³⁺O₃M²⁺O, where M²⁺ is a divalent metal ion. If the metal ion (M²⁺) in the crystal structure is ferrous iron (Fe²⁺), the iron oxide particle is magnetic. When crystal-containing regions of unpaired spins are sufficiently large that they can be regarded as thermodynamically independent, superparamagnetism can occur. These single-domain particles are called magnetic domains. A magnetic domain has a net magnetic dipole which is larger than the sum of the individual unpaired electrons. With no applied magnetic field the magnetic domains are free to rotate and are randomly oriented with no net magnetic field. If an external magnetic field is applied, the magnetic dipoles change their orientation analogous to paramagnetic materials.

A variety of current MRI probes are in the form of paramagnetic complexes or superparamagnetic nanoparticles. There are several ways to synthesize iron oxide nanoparticles, namely, co-precipitation, microemulsion, thermal decomposition or hydrothermal synthesis. A well-established method to synthesize monodisperse Fe₃O₄ (magnetite) particles is described in Sun et al., J. Am. Chem. Soc., 2004, 126, 273-279 which is incorporated herewith by reference for further details. According to this process, oleylamine and oleic acid are used to reduce Fe(acac)₃ into 5 nm sized spheres.

If SPIO particles are encapsulated in polymeric functionalized nanoparticles, the responsive hydrophobic polymer particles will be degraded by the analytes to be detected. Due to this degradation various effects occur at the same time, which all have influence on the relaxation behavior of the water protons. As the polymer matrix degrades, water is penetrating more into the nanoparticle and the distance from the encapsulated SPIO's to the water starts getting smaller, and therefore the influence of the magnetic fields increases on the nearby protons. The magnetite nanoparticles are not soluble in water and therefore form aggregates. This will change the magnetic moment of the magnetite and also decrease the metal surface which limits the area where protons can relax faster, and thus lead to a relaxation change. The relaxation changes can be followed by measuring the T₂ relaxation with NMR spectroscopy.

SPIO contrast agents are commercially available from a number of suppliers. Nanoparticulate SPIO products are sometimes also referred to as USPIO.

Another group of contrast agents which may be exemplary mentioned as component b) of the compositions of the present invention are Gd chelates, which are also available as micro or nanoparticles. Respective products are known to the skilled person and available from a number of commercial suppliers.

In the compositions of the present invention, the MRI contrast agent is embedded or encapsulated in a hydrophobic polymer matrix which restricts the access of water to the contrast agent. The hydrophobic polymer matrix (component a) of the compositions of the present invention) has elements that are sensitive to the analytes selected from the group consisting of reactive oxygen species, proteases or enzymes and when exposed to these analytes, the polymer matrix degrades and water from the surrounding tissue can now gain access to the inside of the nanoparticle. This exposes the encapsulated contrast agent to water effecting changes to the relaxation of water which are then monitored by magnetic resonance imaging.

Methods for embedding or encapsulating micro- or nanoparticulate particles in a polymer matrix are known to the skilled person and have been described in the literature so that no further details are necessary here.

The compositions of the present invention may comprise, as optional component, a functionality capable of binding a marker or a probe or a probe for creating a second detection signal. By including such functionality, complementary dual imaging technologies may be used to increase the amount of information.

There are several ways to include respective functionalities. In accordance with a first variant, the functionality is attached to the responsive hydrophobic polymer a) or, if present, a second non-responsive polymer c) through a pendant functional group preferably selected from the group consisting of amine, thiol, azide or alkyne groups. Respective functional groups as well as methods for their incorporation into polymer matrices are known to the skilled person and have been described in the literature. A number of products are available as commercial products from a variety of suppliers. These functional groups may then be used to attach the probe, e.g. a fluorescent dye to the polymer which then allows to use fluorescence detection methods to be combined with MRI. The fluorescent dye may be attached to the polymer from the beginning or the fluorescent dye may be added to the system during measurement in which case the functional groups described covalently bind the fluorescent dye.

It is also possible, however, to introduce the fluorescent dyes by the use of comonomers which comprise the fluorescent unit, a polymerizable group and one of the functional groups discussed above in the molecule. By using comonomers the fluorescent dye is incorporated into the polymer chain via the polymerizable unit. Suitable polymerizable and functionalized fluorescent dyes are described i.a. in Liu et al., Macromolecules 43, No. 20, 2010, 8315-8330 in scheme 4 on page 8318 to which reference is made herewith for further details. By using these polymerizable fluorescent dyes the fluorescence functionality is introduced into the polymer in the course of polymerization thus avoiding the need for a separate step to attach the probe.

It is readily apparent to the skilled person that fluorescent dyes are only one example for probe c) which may be present in the invention. Other probes, which are suitable to create a signal which may be monitored or determined by other techniques are also suitable.

In one embodiment in accordance with the present invention, the probe suitable for creating a second detectable signal is attached to the polymer a) of the composition as pendant group, i.e. a respective monomer comprising the probe functionality and a polymerizable group is included in the monomer composition used for synthesizing the responsive hydrophobic polymer forming component a) of the compositions of the present invention.

The functional group may also be attached as a pendant group of a second polymer d) which may be present in the compositions in accordance with the present invention as described hereinafter. This second polymer is non-reactive with the analyte under the conditions the responsive hydrophobic polymer a) is capable of undergoing a chain-breaking reaction with the analyte.

By combining a responsive hydrophobic polymer a) with a non-responsive (non-reactive) polymer d) the sensitivity of the composition of the present invention to the analyte to be detected can be tuned through the ratio of polymer a) to the non-reactive second polymer. Thus, in accordance with another embodiment of the present invention, the compositions contain a non-responsive polymer d) in addition to polymer a).

In principle any type of polymer which does not react with the analyte under the conditions of measurement may be used. Respective polymers are known to the skilled person and he will select an appropriate polymer based on his professional knowledge and the requirements of the specific application case.

A preferred second polymer is derived from poly(hydroxy acids) and particularly preferred are poly(lactic-co-glycolic acid polymers), hereinafter referred to as PLGA. PLGA has been used for a variety of applications in vivo, e.g. for the delivery of therapeutics such as small molecular drugs, nucleic acids, peptides and proteins. Because of their magnificent biocompability and biodegradability they are suitable for delivering drugs with sustained release with a single administration.

PLGA is usually synthesized by means of random ring-opening copolymerization of two different monomers, the cyclic dimers (1,4-dioxane-2,5-diones) of glycolic acid and lactic acid. Common catalysts used in the preparation of this polymer include tin(II) 2-ethylhexanoate, tin(II) alkoxides, or aluminum isopropoxide. During polymerization, successive monomeric units (of glycolic or lactic acid) are linked together in PLGA by ester linkages, thus yielding a linear, aliphatic polyester as a product.

Depending on the ratio of lactide to glycolide used for the polymerization, different forms of PLGA can be obtained: these are usually identified in regard to the monomer ratio used (e.g. PLGA 75:25 identifies a copolymer whose composition is 75 mol% lactic acid and 25 mol% glycolic acid). All PLGAs are amorphous rather than crystalline and show a glass transition temperature in the range of 40-60 °C. Unlike the homopolymers of lactic acid (polylactide) and glycolic acid (polyglycolide) which show poor solubilities, PLGA can be dissolved by a wide range of common solvents, including chlorinated solvents, tetrahydrofuran, acetone or ethyl acetate.

If non-reactive polymer d), e.g. PLGA, is present in the compositions of the present invention, the weight ratio of responsive hydrophobic polymer a) to non-responsive polymer d) is not subject to limitations and the weight ratio may be used to tune the selectivity of the composition for a given analyte. As the contrast agent is embedded or encapsulated in polymer a) the amount of this polymer has to be sufficient to achieve the required encapsulation.

The weight ratio of polymer a) to polymer d) usually is in the range of from 90:10 to 20:80, preferably in the range of from 90:10 to 30:70.

The compositions of the present invention are used for the detection and monitoring of analytes through MRI in vivo and in vitro, especially preferably in vivo. In accordance with a preferred embodiment of the present invention the compositions are used in multicellular organisms.

Another embodiment of the present invention relates to a process for the MRI detection of an analyte selected from the group consisting of reactive oxygen species, proteases and enzymes, comprising adding a composition comprising
a) a matrix material based on a responsive hydrophobic polymer capable of undergoing a chemical reaction with the analyte to be detected, such reaction leading to a disruption of the polymer chain of the responsive polymer
b) a contrast agent suitable for use in magnetic resonance imaging, embedded in or encapsulated in the polymer a),
c) optionally, a functionality capable of binding a marker or probe or a probe for creating a second detection signal, and
d) optionally, a non-responsive polymer not undergoing a chemical reaction with the analyte under the conditions where polymer a) undergoes a reaction leading to chain breakage to a system comprising the analyte to be detected and monitoring a magnetic resonance imaging signal.

Preferably the composition is added to a multicellular organism in vivo.

In accordance with another embodiment of the present invention, the compositions are used as *ex vivo* detection kits for the analytes. *Ex vivo* means that the detection process takes place outside an organism. Thus, *ex vivo* refers to experimentation or measurements done in or on tissue in an artificial environment outside the organism with the minimum alteration of natural conditions. *Ex vivo* conditions allow experimentation under more controlled conditions than is possible in *in vivo* experiments (in the intact organism), at the expense of altering the "natural" environment to an extent as small as possible. The peak broadening as a result of the change in relaxation time can be plotted vs the concentration of the analyte, in a similar manner as e.g. in an ELISA plot. Such measurements can basically be carried out with standard NMR equipment.

The compositions allow the detection and monitoring of the level of analytes in tissue environments which are indicative of abnormal physiological conditions or diseases.

In particular the compositions of the present invention allow to detect proteases and enzymes and reactive oxygen species associated with e.g. inflammation processes using MRI.

The compositions of the present invention may thus be used as functional imaging agents to monitor biological processes with MRI techniques.

The following examples show preferred embodiments of the present invention.

**Example 1 - Synthesis of peroxalate polymer**

4-hydroxybenzyl alcohol and 1,8 octanediol (molar ratio 6.6:1) were dissolved in dry tetrahydrofuran (THF) under argon. Triethylamine 40 mmol was added dropwise at 0°C. The mixture was added to 2.4 mmol (0.35g) g of oxalyl chloride in dry THF at 0°C. The reaction mixture was kept at room temperature overnight for 16 hours. Thereafter the reaction mixture was quenched with saturated brine solution and extracted three times with ethyl acetate. The combined organic layers were dried with magnesium sulfate and concentrated under vacuum. The polymer was purified by precipitation (three times) in a mixture of dichloromethane and hexane (volume ratio 1:1). The molecular weight of the product, determined by gel permeation chromatography, was 6200 g/mol and the polydispersity index was 1.83.

**Example 2 - Synthesis of peroxalate polymer comprising functional group for second probe**

The method of example 1 was repeated but 1 mol% of the 4-hydroxybenzyl alcohol was replaced by octapamine

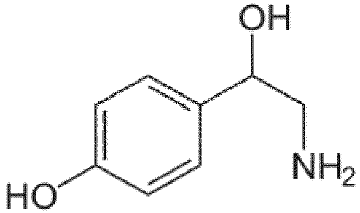

which provided pendant amine functional groups attached to the polymer backbone. A fluorescent tag was attached to the amine group thus allowing to monitor a fluorescence signal with near infrared tomography.

**Example 3 - Synthesis of MRI sensitive Fe₃O₄-Peroxalate nanoparticles**

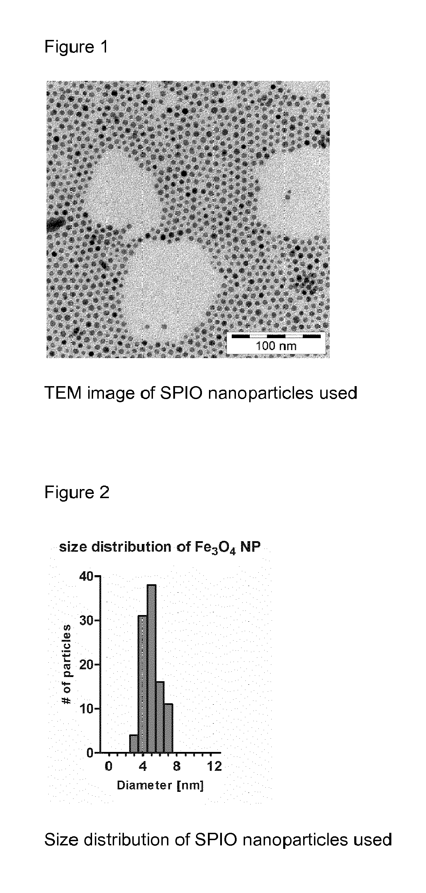

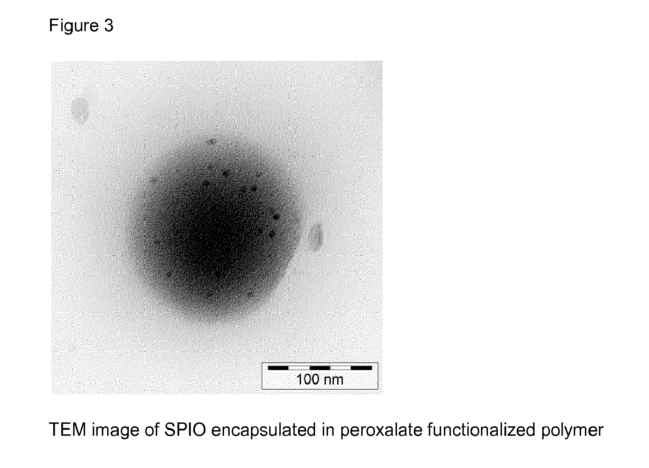

The sensitive nanoparticles were synthesized by single step nanoprecipitation. The Fe₃O₄ nanoparticles (TEM image and size distribution are shown in Figs. 1 and 2, respectively) were dissolved in a concentration in the range of from 0.1 to 0.2 mmol/L in added together with 0.5 mg of peroxalate polymer and 0.5 mg of a PLGA polymer (lactic:glycolic acid molar ratio 50:50, weight average molecular weight 24,000-38,000 g/mol. The functional NP's were formed by adding H₂O into THF. The formed nanoparticles were then worked up by dialysis in water. The size of the NP's was determined with TEM (cf. Figure 3) and Dynamic Light Scattering (DLS). In the TEM image the dark round spots are magnetite particles which absorb more electromagnetic radiation than the polymer scaffolding which appears less dark. The average size of the functional nanoparticles measured with DLS was 150 nm.

**Example 4 - MRI measurements**

The relaxation measurements were conducted with a *Bruker Avance 300* NMR spectrometer. To determine the T₁ and T₂ relaxation rates of the ironoxide nanoparticles, different concentrations of SPIO were dissolved in CHCl₃/CDCl₃. For T₂ a Carr-Purcell-Meiboom-Gill (CPMG) sequence was applied and the Free Induction Decay (FID) of the solvent protons was followed. For the functionalized nanoparticles the solvents for the T₂ measurements were a mixture of H₂O and D₂O.

With the software *Top Spin 3.1* (Bruker) the data was processed. The T₂ times were calculated with *Biospin, Dynamics Center 2.0.4* (Bruker) and *Origin 9.0G.*

The measurements of magnetite were conducted at 293 K and the measurements of the functional nanoparticles with hydrogen peroxide at 300 K

a) *Relaxation rate of Fe₃O₄ nanoparticles*

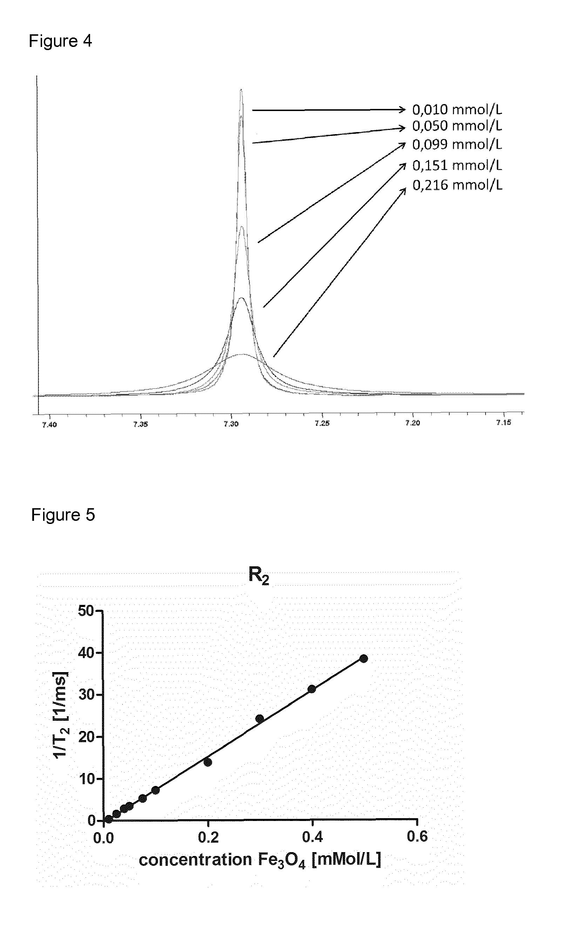

The magnetite was dissolved in CHCl₃/CDCl₃. Afterwards several concentrations were diluted. The samples were put into NMR tubes and measured at 293 K. The ¹H spectra (cf. Fig. 4) show a peak broadening with higher concentrations of Fe₃O₄ which points out the T₂ effect of the nanoparticles on the solvent protons.

After the measurements the CPMG experiments were processed (Fourier transformed and phase corrected) with *Top Spin.* The processed data file (2rr) was opened in *Dynamics Center* and T₂ was calculated. With *GraphPad Prism 5* T₂ was plotted against the concentration of magnetite with (cf. Figure 5).

The r₂ relaxation value was calculated from the slope of the linear plots of 1/T₂ versus the magnetite concentrations with *Origin* (80 L*mMol⁻¹*s⁻¹).

*b) Relaxation of MRI sensitive iron-oxide-peroxalate polymer nanoparticles*

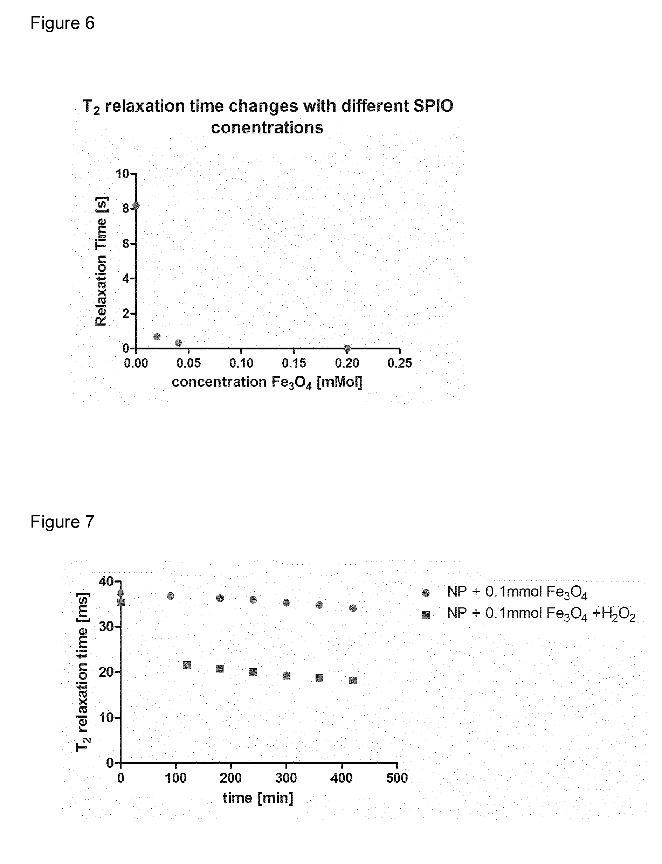

The nanoparticles were precipitated in THF with water. The concentration of magnetite nanoparticles was varied from 0 to 0.2 mMol. After dialysis in water the nanoparticles were filled in NMR tubes with D₂O. The results of the relaxation time measurements are shown in Figure 6.

c) *Relaxation change of MRI sensitive iron-oxide-peroxalate polymer nanoparticles with H₂O₂*

Two samples (A + B) were prepared with peroxalate polymer and 0.05 mMol Fe₃O₄ nanoparticles. The T₂ relaxation time of both samples was determined first. In Sample B H₂O₂ was added and sample A was used as a control. The relaxation time of both samples was observed over 420 minutes. There is a drastic change in T₂ when H₂O₂ was added as shown in Figure 7. The relaxation time of sample A without H₂O₂ is decreasing only slowly, reflecting the normal degradation of hydrogen peroxide in water.

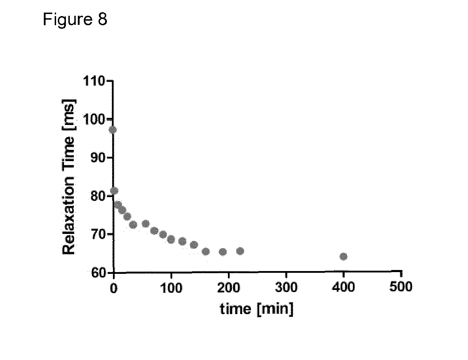

In Figure 8 the relaxation time decrease after adding H₂O₂ into a hydrogen peroxide sensitive probe is observed more closely. A reasonable decrease can be seen immediately with the first measurement after the addition of H₂O₂. After 200 minutes the decay attenuates. This endorses a very sensible H₂O₂ probe since the biggest change in relaxation change is immediately after addition. The change of the relaxation time is roughly 35% in total.

## Claims

1. Injectable compositions for the MRI detection of an analyte selected from the group consisting of reactive oxygen species, proteases and enzymes, comprising
a) a matrix material based on a responsive hydrophobic polymer capable of undergoing a chemical reaction with the analyte to be detected, such reaction leading to a disruption of the polymer chain of the responsive polymer,
b) a contrast agent suitable for use in magnetic resonance imaging, embedded in or encapsulated in the polymer a),
c) optionally, a functionality capable of binding a marker or probe or a probe for creating a second detection signal, and
d) optionally, a non-responsive polymer not undergoing a chemical reaction with the analyte under the conditions where polymer a) undergoes a reaction leading to chain breakage.

2. Compositions in accordance with claim 1, wherein the contrast agent is selected from T₁ and T₂ contrast agents.

3. Compositions in accordance with claim 1 or 2 wherein the polymeric matrix material comprises units of formulae (1) and/or (2) in the polymer chain

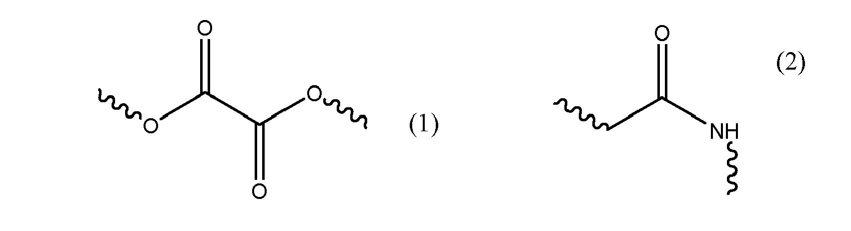

4. Compositions in accordance with any of claims 1 to 3 wherein the functionality c) is selected from amine, thiol, azide or alkyne groups.

5. Composition in accordance with any of claims 1 to 3 wherein the probe c) is a fluorescent dye.

6. Compositions in accordance with any of claims 1 to 5 comprising a poly(hydroxy acid) polymer as component d).

7. Compositions in accordance with claim 6 wherein the polymer d) is a copolymer of lactic acid and glycolic acid (PLGA).

8. Composition in accordance with any of claims 1 to 7 wherein the contrast agent b) is selected from Gd-chelates and superparamagnetic iron oxide (SPIO) particles.

9. A process for the MRI detection of an analyte selected from the group consisting of reactive oxygen species, proteases and enzymes, comprising adding a composition comprising
a) a matrix material based on a responsive hydrophobic polymer capable of undergoing a chemical reaction with the analyte to be detected, such reaction leading to a disruption of the polymer chain of the responsive polymer
b) a contrast agent suitable for use in magnetic resonance imaging, embedded in or encapsulated in the polymer a),
c) optionally, a functionality capable of binding a marker or probe or a probe for creating a second detection signal, and
d) optionally, a non-responsive polymer not undergoing a chemical reaction with the analyte under the conditions where polymer a) undergoes a reaction leading to chain breakage
to a system comprising the analyte to be detected and monitoring a magnetic resonance imaging signal.

10. Process in accordance with claim 9 wherein the composition is added to a multicellular organism in vivo.

11. Use of the compositions in accordance with any of claims 1 to 8 for the detection of reactive oxygen species, proteases or enzymes by magnetic resonance imaging.

12. Use in accordance with claim 11 wherein the analytes are detected in vivo.

13. Use in accordance with claim 11 wherein the analytes are detected ex vivo.
